# EUROPEAN PATENT APPLICATION

(11) **EP 1 887 006 A1**
(43) Date of publication of application: **13.02.2008**
(21) Application number: 06016428.2
(22) Date of filing: 07.08.2006
(51) Int. Cl.: C07D 417/12, A61K 31/435, A61P 3/10

(54) **Polymorphic forms of rosiglitazone base**

(71) Applicant: KRKA, 8501 Novo mesto (SI)
(72) Inventor: Zupancic, Silvo, 8000 Novo Mesto (SI); Smrkolj, Matej, 1411 Izlake (SI); German, Tamara, 8000 Novo Mesto (SI); Smodis, Janez, 8000 Novo Mesto (SI); Vrbinc, Miha, 8000 Novo Mesto (SI)
(74) Representative: Becker Kurig Straus

(57) **Abstract**

The present invention relates to novel polymorphic forms of rosiglitazone base, as well as to a process for their preparation and pharmaceutical formulations containing it. The present polymorphic forms of rosiglitazone base are characterized by small average particle sizes and high solubility bestowing the compounds an improved bioavilability.

## Description

### Field of the invention

The present invention relates to novel polymorphic forms of rosiglitazone base, as well as to a process for their preparation and pharmaceutical formulations containing it. The present polymorphic forms of rosiglitazone base are characterized by small average particle sizes and high solubility bestowing the compounds an improved bioavilability.

### Background of the invention

Rosiglitazone is the common name for 5-[4-[2-(N-methyl-N-(2-pyridyl)amino)ethoxy]-benzyl]-2,4-thiazolidinedione referred to as rosiglitazone base, a compound of formula (I):

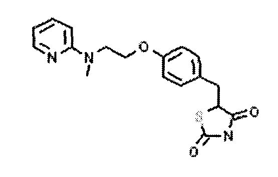

Rosiglitazone belongs to the thiazolidinedione class and exhibits anti-diabetic activities and is particularly used for the treatment of non-insulin dependent diabetes (NIDDM). Like other thiazolidinediones, its mechanism of action is by activation of the intracellular receptor class of the peroxisome proliferator-activated receptors (PPARs), specifically peroxisome proliferator-activated receptor gamma (PPARγ). Apart from rosiglitazone's effect on insulin resistance, anti-inflammatory effects were also observed (Mohanty et al.; J. Clin. Endocrinol. Metab.; Jun;89(6) (2004) 2728-35.). Use of rosiglitazone and compositions thereof are for example disclosed in US 5,002,953, US 5,741,803. WO 94/05659 describes inter alia subsituted rosiglitazone maleates.

Commercial forms of rosiglitazone are different crystalline salts, particularly rosiglitazone maleate, due to an improved stability and solubility in water in comparision to rosiglitazone base.

The above characteristics are further influenced by the conformation and orientation of molecules in the unit cell defining a particular polymorphic form of a substance. These conformational and orientational factors in turn result in particular intramolecular interactions and intermolecular interactions with adjacent molecules that influence the macroscopic properties of the bulk compound. A particular polymorphic form may give rise to distinct spectroscopic properties that may be detectable by powder X-ray diffraction, solid state 13 C NMR spectrometry and IR spectrometry. The polymorphic form may also give rise to thermal behaviour different from that of the amorphous material or another polymorphic form. Thermal behaviour is measured in the laboratory by such techniques as capillary melting point, thermo gravimetric analysis (TGA) and differential scanning calorimetry (DSC) and may be used for distinguishing polymorphic forms.

The discovery of new polymorphic forms of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

WO 2004/085435, WO 2006/010653 and GB2421240 disclose polymorphic forms of rosiglitazone maleate and rosiglitazone posphoric salts, respectively. Such polymorphic forms are of particular interest due to the provision of essentially pure compounds permitting an exact characterization of physico-chemical properties and possible favourable pharmocological characteristics, such as improved solububility and particle size.

There is, however, still a need for rosiglitazone forms and/or compositions therof which display improved solubility, particularly over the full pharmacological pH range.

### Object of the invention

Accordingly an object of the present invention resides in the provision of other polymorphic forms of rosiglitazone salts, exhibiting desired pharmcological properties for the preparation of pharmaceutical compositions.

The present invention solves the above-mentioned problems by the provision of polymorphic forms I, II and III of rosiglitazone base, characterized by respective X-ray powder diffraction (XRD) peaks, respective methods for their preparation and the use of said polymorphic forms in pharmaceutical compositions.

During the extensive studies leading to the present invention it has been unexpectedly found that allready rosiglitazone base may be provided in different polymorphic forms (I, II and III) suitable for the preparation of pharmaceutical compositions. Said polymorphic forms exhibit improved pharmocological properties, particularly an enhanced water soubiltity, in comparision to conventional rosiglitazone. The polymorphic forms disclosed exhibit further similar or even enhanced pharmocological characteristics in comparision to allready known polymorphic forms of rosiglitazone salts. A further advantage resides in a reduced molecular weight of rosiglitazone base in comparision ro rosiglitazone salts permitting administration forms, e.g. tablets, of smaller volume containg, however, the same amount of active ingredient(s). In addition, the preparation of said polymorphic forms of rosiglitazone base is facilitated since respective reaction steps otherwise required for the preparation and purification of rosiglitazone salts are omitted. Another advantage resides in that the omission of salts, such as maleate, permits the preparation of pharmaceutical compositions suitable for the treatment of patients exhibiting an intolerability, such as an allergy, towards a particular salt.

### Figures

Figure 1 shows an XRD spectrum (a), the respective data table (b) and microscope picture (c) of rosiglitazone base polymorphic form I.

Figure 2 shows an XRD spectrum (a), the respective data table (b) and microscope picture (c) of rosiglitazone base polymorphic form II.

Figure 3 shows an XRD spectrum (a), the respective data table (b) and microscope picture (c) of rosiglitazone base polymorphic form III.

### Detailed description of the invention

According to a first embodiment of the present invention polymorphic forms I, II and III of rosiglitazone base are provided.

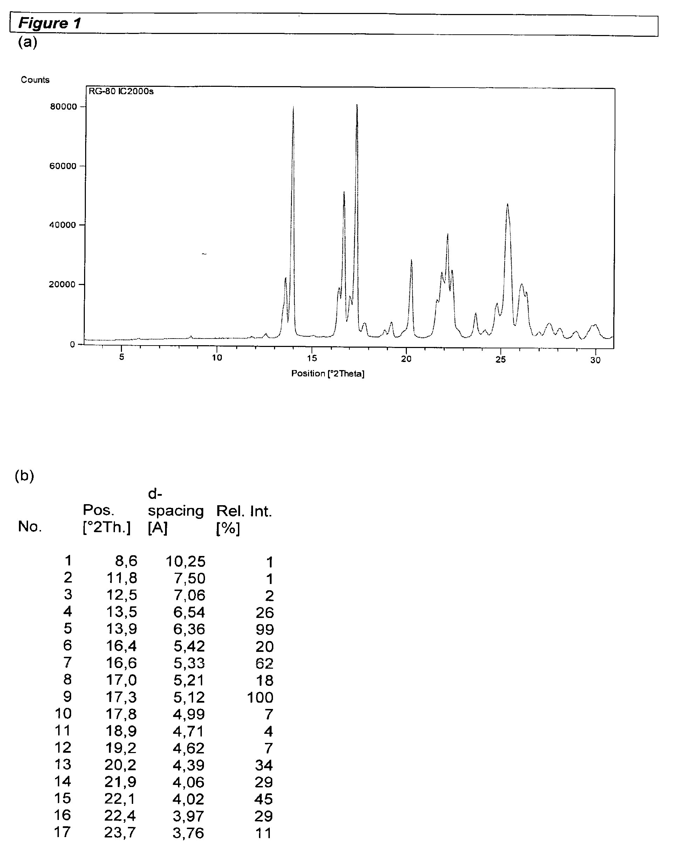

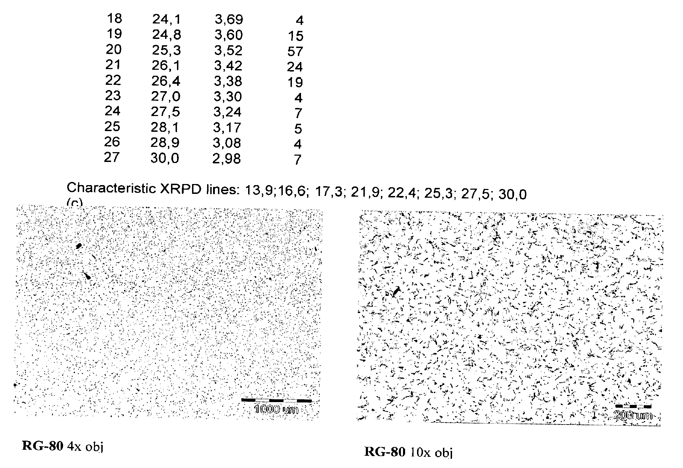

Polymorphic form I is characterized by a X-ray powder diffraction pattern exhibiting characteristic diffractions at 13,9; 16,6; 17,3; 21,9; 22,4; 25,3; 27,5 and 30,0° ± 0,2°2Theta. Preferably form I is characterized by a X-ray powder diffraction pattern exhibiting characteristic diffractions as outlined in figure 1.

Polymorphic form I is further characterized by a melting point in the range of form 153-156°C, preferably in the range of from 153-154°C.

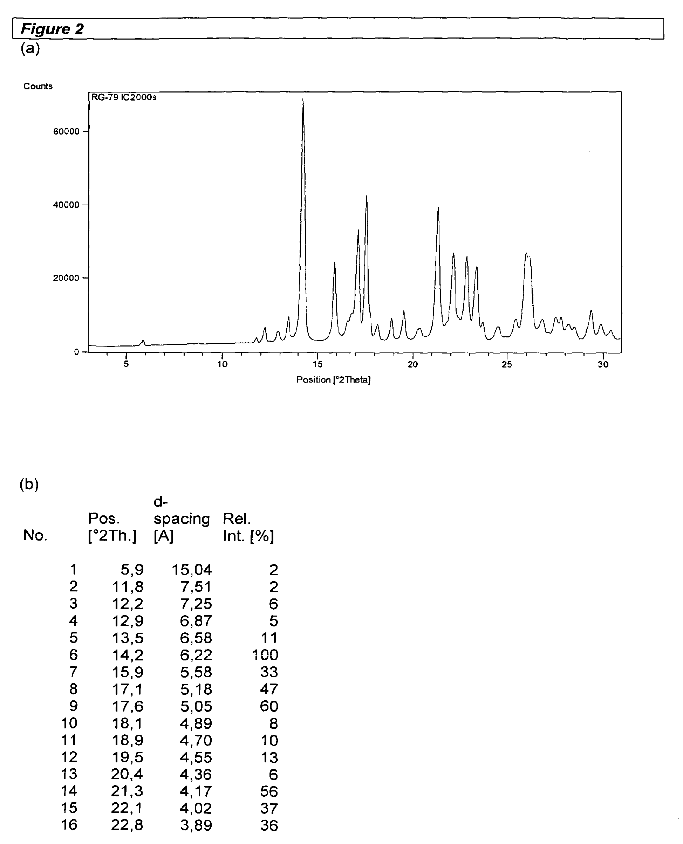

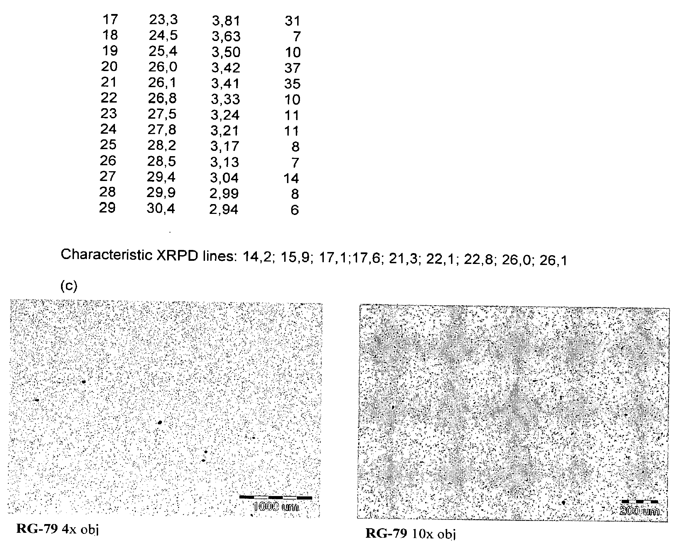

Polymorphic form II is characterized by a X-ray powder diffraction pattern exhibiting characteristic diffractions at 14,2; 15,9; 17,1;17,6; 21,3; 22,1; 22,8; 26,0 and 26,1° ± 0,2°2Theta. Preferably form II is characterized by a X-ray powder diffraction pattern exhibiting characteristic diffractions as outlined in figure 2.

Polymorphic form II is further characterized by a melting point in the range of form 151-154°C, preferably in the range of from 151-152°C.

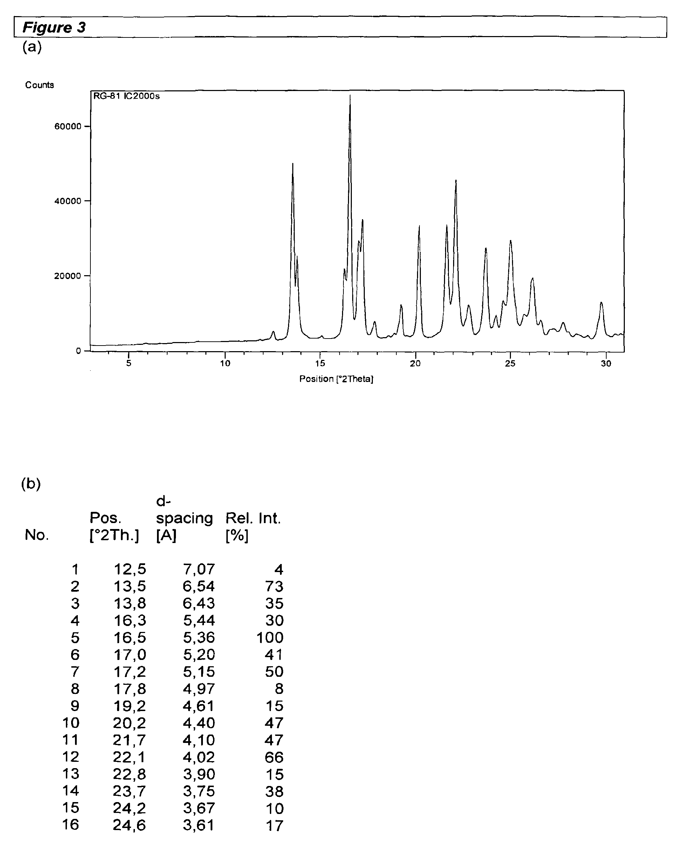

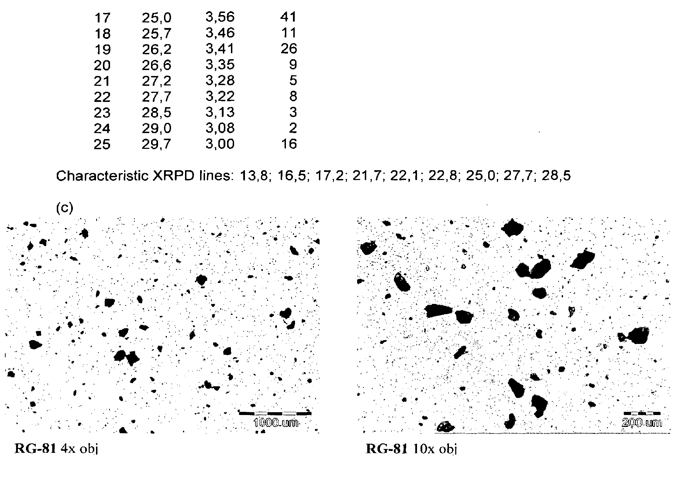

Polymorphic form III is characterized by a X-ray powder diffraction pattern exhibiting characteristic diffractions at 13,8; 16,5; 17,2; 21,7; 22,1; 22,8; 25,0; 27,7 and 28,5° ± 0,2°2Theta. Preferably form III is characterized by a X-ray powder diffraction pattern exhibiting characteristic diffractions as outlined in figure 3.

Polymorphic form III is further characterized by a melting point in the range of form 140-149°C, preferably in the range of from 144-145°C.

The polymorphic form I, II or III are further characterized by a small particle size between 1 and 200 µm, preferably 5-100 µm, most preferably 10-40 µm, which may be determined by a laser method using a Malvern Mastersizer.

Said polymorphic forms I, II and III of rosiglitazone base may be present in the form of hydrates or solvates and favour inter alia due to the small particle size a high bioavailability - representing a measure of the rate and extent of a therapeutically active drug that reaches the systemic circulation and which is available at the site of action - of the present compound.

It should be clear that also rosiglitazone salts of polymorphic forms I, II and III of rosiglitazone base are encompassed. Polymorphic forms I, II and III of rosiglitazone base could be used for the preparation of suitable salts such as maleate, hydrochloride and oxalate. Said salts may be obtained for example by adding maleic acid, HCl, oxalic acid to the polymorphic forms I, II and III of rosiglitazone base in a manner well known to the skilled person.

According to another preferred embodiment of the present invention, a process for the preparation of compounds according to the present invention is provided.

The process for the preparation of polymorphic form I of rosiglitazone base comprises (i) dissolving rosiglitazone base in an organic solvent at a temperature in the range of from 20°C to reflux temperature of said organic solvent; (ii) optionally adding an anti-solvent; (iii) cooling to a temperature in the range of from -10°C to 30°C and (iv) separating and drying of Polymorphic form I of rosiglitazone base. The organic solvent used is selected from the group consisting of an alcohol, an ester, an ether, a ketone and tetrahydrofurane (THF), preferably the organic solvent is methanol, methyl-acetate, ethyl-acetate or isopropyl acetate, more preferably the organic solvent is methanol. The anti-solvent is directed to any solvent capable to precipitate rosiglitazone base from the chosen organic solvent and is selected from the group consisting of water and an organic antisolvent, such as an ether or toluene. Alternatively, polymorphic form I of rosiglitazone base my be prepared by a process comprising the steps of (i) dissolving rosiglitazone base in an organic solvent at a temperature in the range of from 20°C to reflux temperature of said organic solvent; (ii) optionally adding water and (iii) lyophilising and drying. The organic solvent is selected from the group consisting of an alcohol, an ester and a ketone, preferably the organic solvent is methanol, methyl-acetate, ethyl-acetate or isopropyl acetate. Cooling is hereby performed at a slow rate as may be achieved by leaving the vessel containing rosiglitazone base in a refrigerator at either 4-8°C or 20°C. A slow rate of cooling is generally intended to comprise rates of below 1°C/min..

The process for the preparation of Polymorphic form II of rosiglitazone base comprises the steps of (i) dissolving rosiglitazone base in an organic solvent at a temperature in the range of from 20°C to reflux temperature of said organic solvent; (ii) adding an anti-solvent and cooling to a temperature in the range of from -10°C to 30°C or cooling to a temperature in the range of from -10°C to 30°C at a rate of 1 to 30 °C/min. and stirring and (iii) separating and drying of Polymorphic form II of rosiglitazone base. The organic solvent is selected from the group consisting of tetrahydrofurane, dioxane, an alcohol, an ester and a ketone, preferably the organic solvent is methanol, tetrahydrofurane, methyl-acetate, ethyl-acetate or isopropyl acetate, more preferably the organic solvent is methanol or tetrahydrofurane. The anti-solvent is selected from the group consisting of an organic antisolvent, such as alkanes or cycloalkanes, preferably the organic solvent is heptane, hexane or cyclohexane.

Alternatively, polymorphic form II of rosiglitazone base may be obtained by a process comprising the steps of (i) dissolving rosiglitazone base in an organic solvent at a temperature of from 20°C to reflux temperature of said solvent; and (ii) evaporating said organic solvent under normal pressure or reduced pressure. The organic solvent used is a ketone.

The process for the preparation of Polymorphic form III of rosiglitazone base comprises the steps of (i) dissolving rosiglitazone base in an aqueous solution of an inorganic base at a temperature in the range of from 20°C to 100°C; (ii) adding an aqueous solution of an inorganic acid until neutralization; (iii) stirring at a temperature in the range of from 0°C to 20°C and (iii) separating and drying of Polymorphic form III of rosiglitazone base. Examples for a suitable inorganic base comprise KOH, NaOH or LiOH, whereas examples for a suitable inorganic acid comprise capable of neutralizing comprise HCl, H₂SO₄, HNO₃, HClO₄, HBr and HI. Neutralizing is hereby intended to adjust the pH to approximate neutrality in the range of from pH 6.7 to 7.3. Alternatively, Polymorphic form III of rosiglitazone base may be obtained by a process comprising the steps of (i) dissolving rosiglitazone base in an organic solvent at a temperature in the range of from 20°C to reflux temperature of said organic solvent; (ii) and evaporating said organic solvent under normal pressure or reduced pressure. The organic solvent used is selected from the group consisting of an alcohol, an ester, an ether and tetrahydrofurane (THF), preferably the organic solvent is methanol, methyl-acetate, ethyl-acetate or isopropyl acetate, more preferably the organic solvent is methanol. The term reduced pressure relates to a pressure reduced in comparison to the normal (ambient) pressure. The temperature during evaporation may be in the range of from -10°C to 60°C, preferably in the range of from 0°C to 20°C.

Rosiglitazone base (also named crude or free rosiglitazone base) as educt for the respective processes for the preparation of the polymorphic forms I, II and III of rosiglitazone base may be prepared according to any method known to the skilled person. The preparation of rosiglitazone base is outlined for example in EP 0 306 228.

The reflux temperature of a solvent as used herein relates to a temperature in the range of the boiling temperature of a particular solvent to the boiling temperature plus 15°C, i.e. the reflux temperature of methanol is in the range of from 78°C to 93°C.

Separating the polymorphic form I, II or III of rosiglitazone base may be performed according to any technique known to the skilled person, for example filtration. Drying as used herein refers to the removal of solvent residues performed for example by heating or vacuum.

All of the above mentioned processes for the preparation of polymorphic form I, II or III of rosiglitazone base yield surprsingly very small, homogenous particles (as may be seen from the photographs of the different polymorphic forms), which are suitable to be incorporated directly into the formulation. This provides for further advantage since no complicated grinding, milling or micronisation is required. The particle size is extraordinarily important for pharmaceutical use of the product and it is often very labor-intensive or impossible to obtain a product that would have a suitable size of particles. Methods of manufacture that produce such a product directly are, therefore, unique and exceptionally desirable.

According to another preferred embodiment of the present invention pharmaceutical compositions or formulations comprising polymorphic form I, II or III of rosiglitazone base or its pharmaceutically acceptable salts as an active ingredient are encompassed. The use of polymorphic form I, II or III of rosiglitazone base or its pharmaceutically acceptable salts provides hereby the advantage that a surfactant in the composition may be omitted and optionally substituted by a disintegrant, or by addition of an acid to the formulation to increase solubility and dissolution. Of particular advantage is that the present polymorph forms may be used directly in the preparation of pharmaceutical formulations without the need of a conversion to the salt giving raise to a more economical process having less steps and that lower amounts of reagents, such as solvents or acids, are required. Using the base provides the possibility to omit unnecessary ingredients (such as an acid), which have to be otherwise ingested, and to reduce the weight of the active ingredient. In addition, the present polymorphic forms I, II or III of rosiglitazone base provide improved stability in comparisons to amorphous forms, in that stabilizers may be omitted.

The pharmaceutical compositions are preferably solid pharmaceutical compositions comprise polymorphic form I, II or III of rosiglitazone base together with excipients. Rosiglitazone base may be for example introduced into the pharmaceutical formulation in its crystalline form (i.e. anhydrous) or hydrated.

The pharmaceutical compositions of the present invention can be in the form of tablets, pills, powders, lozenges, sacchets, soft and hard gelatine capsules, suppositories etc. The dosage form is preferably suitable for oral application.

The compositions are preferably formulated in a unit dosage form, each dosage containing about 1 to 100 mg, more usually 1 to about 16 mg of rosiglitazone. The term "unit dosage form" refers to physically discrete units suitable as unitary dosages for human objects and other mammals, each unit containing a predetermined quantity of rosiglitazone calculated to produce the desired therapeutic effect, in association with a suitable pharmaceutical excipient.

In a further preferred embodiment the composition according to the invention is such that the average particle size of rosiglitazone is between 1 and 200 µm, preferably 5-100 µm, most preferably 10-40 µm. The average particle size may be determined by a laser method using a Malvern Mastersizer.

The excipients used for the preparation of the pharmaceutical formulations according to the present invention particularly include for example diluents, binders, disintegrants, lubricants and acidic agents. Other and further excipients can also be contained and the opposite, one ore more excipient can be excluded form the composition.

The excipients present in the composition according to the invention may be diluents such as microcrystalline cellulose, powdered cellulose, lactose (anhydrous and monohydrate), compressible sugar, fructose, dextrates, sugar alcohols such as mannitol, sorbitol, maltitol, xylitol, lactitol, or other sugars such as saccharose, raffinose, trehalose, fructose or mixture thereof, siliconised microcrystalline cellulose, calcium hydrogenphosphate, calcium carbonate, calcium lactate or mixture of diluents. Preferably, excipients include at least one excipient, selected from microcrystalline cellulose, lactose and mannitol, or mixtures thereof.

The composition according to the invention can also comprise binders, such as polyvinylpyrrolidone, microcrystalline cellulose, hydroxyethylcellulose, hydroxypropylcellulose, hydroxypropylmethylcellulose or other cellulose ethers, starch, pregelatinised starch, or polymethacrylate, or mixtures thereof. It is preferable to use a binder with good water solubility. In view of the practical insolubility of starch in cold water, there is a strong preference for binders with a very good solubility in cold water. There is a variety of binders that have very good solubility in water, e.g. povidone of different K-values, hydroxypropylmethylcellulose and hydroxypropylcellulose, i.e. partially substituted poly(hydroxypropyl) ether of cellulose and/or low substituted poly(hydroxypropyl) ether of cellulose.

Further, the pharmaceutical compositions of the present invention can also contain disintegrants, such as crospovidone, pregelatinised starch, sodium strach glycollate, microcrystalline cellulose, carboxymethylcellulose sodium (CMC-Na) or calcium (CMC-Ca), cross-linked CMC.Na, polacrilin potassium, low-substituted hydroxypropylcellulose or mixtures thereof. Preferably, at least one disintegrant is selected from sodium strach glycollate, cross-linked CMC-Na and low-substituted hydroxypropylcellulose.

The composition according to the present invention can further comprise lubricants, such as stearic acid or stearic acid salts, such as e.g. magnesium stearate, magnesium palmitate, magnesium oleate, hydrogenated vegetable oil, hydrogenated castor oil, talc, sodium stearyl fumarate, macrogols or mixtures thereof. Preferably, excipients include at least one lubricant selected from stearic acid, magnesium stearate, hydrogenated vegetable oil, hydrogenated castor oil and talc.

The composition according to the present invention can finally comprise surfactants. Surfactant molecules may be classified based on nature of the hydrophilic group within the molecule. The four main groups of surfactants are defined as follows: 1. *Anionic surfactants,* where the hydrophilic group carries a negative charge, such as carbonyl (RCOO⁻), sulphonate (RSO₃⁻) or sulphate (ROSO₃⁻). Examples of pharmaceutical importance include potassium laurate, CH₃(CH₂)₁₀COO⁻K⁺, and sodium lauryl sulphate, CH₃(CH₂)₁₁SO₄⁻Na⁺. 2. *Cationic surfactants,* where the hydrophilic group carries a positive charge (e.g., quaternary ammonium halides, R₄N⁺Cl⁻). Examples of pharmaceutical importance include cetrimide, a mixture consisting mainly of tetradecyl (ca. 68%), dodecyl (ca. 22%), and hexadecyltrimethylammonium bromides (ca. 7%), as well as benzalkonium chloride, a mixture of alkylbenzyldimethylammonium chlorides of the general formula [C₆H₅CH₂N⁺(CH₃)₂R]Cl⁻, where R represents a mixture of the alkyls from C₈H₁₇ to C₁₈H₃₇. 3. *Ampholytic surfactants (also called zwitterionic surfactants),* where molecule contains, or can potentially contain, both a negative and a positive charge, (e.g., the sulfobetaines, RN⁺(CH₃)₂CH₂CH₂SO₃⁻). Examples of pharmaceutical importance include *N*-Dodecyl-*N*,*N-*Dimethylbetaine, C₁₂H₂₅N⁺(CH₃)₂CH₂COO⁻. 4. *Nonionic surfactants,* where the hydrophile carries no charge but derives its water solubility from highly polar groups such as hydroxyl or polyoxyethylene (OCH₂CH₂O-) groups. Examples of pharmaceutical importance include polyoxyethylated glycol monoethers (e.g. cetomacrogol), sorbitan esters (Spans^{®}) and polysorbates (Tweens^{®}).

A special example for surfactants are poloxamers, that are defined as nonionic polyoxyethylene-polyoxypropylene copolymers. They are chemically similar in composition, differing only in the relative amounts of propylene and ethylene oxides added during manufacture.

Lubricants, glidants and antiadherents used in solid pharmaceutical compositions of the present invention, are herein not defined as surface-acting agents (surfactants).

Acidic agent for the purposes of the present invention can be also included into formulation. Acidic agent can be selected form the group of pharmaceutically acceptable anorganic or organic acids. Anorganic acids include e.g. hydrochloric, sulphonic, phosphonic acid etc. Organic acid may can be selected form any of organic carboxylic acid, preferably an aliphatic organic carboxylic acid having C₃-C₂₀ carbon atoms, for example, tartaric, malic, succinic, glutaric, glutamic, maleic, mandelic, citric, alginic, asparaginic, stearic, tosylic, besylic, salicylic, lactic, benzoic, acetic acid and the like.

The pharmaceutical composition according to the present invention comprises 0.1-10%, preferably 0.3-7%, more preferably 0.5-5% by weight of rosiglitazone; 1-99%, preferably 10-93%, more preferably 30-95% by weight of diluent; 0.1-10%, preferably 0.3-7%, more preferably 0.5-5% by weight of binder; 0.2-20%, preferably 0.5-15%, more preferably 1-10% by weight of disintegrant; 0.1-10%, preferably 0.1-7%, more preferably 0.1-5% by weight of lubricant, 0.1-5%, preferably 0.2-4%, more preferably 0.3-3% by weight of surfactant, 0.1-5%, preferably 0.2-4%, more preferably 0.3-3% by weight of acidic agent.

The preferred pharmaceutical composition according to the present invention includes at least an active ingredient (i.e. polymorphic form I, II or III of rosiglitazone base or pharmaceutically acceptable salts thereof) and one of the excipients as defined above, i.e. diluent, binder, disintegrant, lubricant, surfactant and acidic agent.

Even more preferred are compositions comprising the above defined preferred excipients. If not indicated otherwise, all percentages given herein are by weight based on the total weight of the composition.

The composition according to the invention is preferably in form of a coated or uncoated tablet, e.g. fast disintegrating tablet or orally disintegrating tablet, a capsule or in form of pellets. The composition can also take the form of a powder mixture, of a granulate or of mini tablets filled in capsules. An immediate release composition is preferred.

Optionally, cores/tablets can be coated with conventional materials used for film coating, i.e. as described in Pharmaceutical Coating Technology (G. Cole (ed.), 1995). Film coating formulations usually contain the following components: polymer(s), plasticizer(s), colourant(s)/opacifier(s), and vehicle(s).

In film coating suspension minor quantities of flavors, surfactants and waxes can be used. The majority of the polymers used in film coating are either cellulose derivatives, such as the cellulose ethers, or acrylic polymers and copolymers. Occasionally encountered are high molecular weight polyethylene glycols, polyvinyl pyrrolidone, polyvinyl alcohol and waxy materials. Their function usually is to prevent bad mouth feel and/or taste and in some cases degradation, e.g. oxidation of the active ingredients and/or excipients used.

Typical cellulose ethers which may be applied as coatings are hydroxyethylcellulose, hydroxypropylcellulose, hydroxypropylmethylcellulose and methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

The commonly used plasticizers can be categorized into three groups: 1. polyols (glycerol, propylene glycol, macrogols); 2. organic esters (phthalate esters, dibutyl sebacetate, citrate esters, triacetin); and 3. oils/glycerides (castor oil, acetylated monoglycerides, fractionated coconut oil).

Colourants/opacifiers are also classified into several groups: 1. organic dyes and their lakes; 2. inorganic colours, and 3. natural colours.

Different materials from each group can also be combined in defined ratios. Film coating suspensions can also be used as ready-to-make preparations which are available on the market.

Film coating dispersion can be prepared by using different solvents (water, alcohols, ketones, esters, chlorinated hydrocarbons), preferably water.

A composition of coating suspension (calculated on dry material) comprising 1-99%, preferably 1-95% by weight of polymer; 1-50%, preferably 1-40% by weight of plasticizer; 0.1-20%, preferably 0.1-10% by weight of colourant/opacifier is particularly preferred.

Conventional equipment for applying a coating, such as a Wurster coating system or conventional coating pans can be used.

The present pharmaceutical formulations are prepared by known technological procedures, e.g. by direct compression, dry or wet granulation, using well known and readily available excipients. In the preparation of the compositions of rosiglitazone, the active ingredient will usually be mixed with an excipient or mixture of excipients, or diluted with an excipient or mixture of excipients. When the excipient serves as a diluent, it may be a solid, solid or liquid material which acts as a vehicle or medium for rosiglitazone.

It has been found out that the inventive solid oral dosage form which is prepared by wet granulation is stable at ICH stability testing conditions. Further, drying a granulate, prepared in a wet granulation process, in a fluid-bed dryer enables the preparation of round-shaped particles of granulate consisting of rosiglitazone and excipient(s), which provide a reproducible and processible formulation.

The wetting of a mixture of rosiglitazone and excipient(s) can be performed in conventional granulation equipment by spraying of water, alcohol, water/alcohol mixture or aqueous, alcoholic or aqueous/alcoholic granulating liquid onto an excipient or mixture of excipients by conventional pharmaceutical techniques. Wetting can also be effected by direct addition of water, alcohol, water/alcohol mixture or aqueous, alcoholic or aqueous/alcoholic granulating liquid to mixture of excipients during mixing operation in a proper mixing device, e.g. high-shear mixer. The terms »aqueous, alcoholic or aqueous/alcoholic granulating liquid« refers to an aqueous, alcoholic or aqueous/alcoholic dispersion which contains purified water or demineralised water or lower alcohols (methanol, ethanol, isopropanol) as diluents and a solid substance which is dispersed, suspended or dissolved in the diluent. The dispersed substance can have known functions of excipients used in the present compositions comprising rosiglitazone, e.g. binder, surfactant or acidic agent.

Mixing of the excipients or of excipients with rosiglitazone may be effected in conventional devices used for mixing of powders, e.g. motionless (passive) mixers, fluidized bed, diffusion, biconic diffusion, uniconic, biconic, turbular, cubic, planetary, Y-, V-shaped or high-shear mixers.

For drying of granulate, conventional drying devices such as a fluid-bed dryer or drying chambers (non-vacuumized or vacuumized) can be used.

In the process as described above, the compression, in particular to cores/tablets, can be effected using an automatic rotary compressing machine form different manufacturers of equipment for use in pharmaceutical industry.

Conventional equipment can be used for applying film coating, such as fluid bed (e.g. Wurster coating system) or conventional coating pans for use in pharmaceutical industry.

The process for preparing the pharmaceutical composition according to the invention can be carried out as a granulation process or direct compression process. In each case, rosiglitazone is first prepared according to a suitable synthetic process and then purified, e.g. by crystallization or other means. Then, the size of rosiglitazone particles is determined and if it is found that there are particles having a suitable particle size, then this rosiglitazone is milled or crunched to a smaller size, i.e. that the average particle size of rosiglitazone is between 1 and 200 µm, preferably 5-100 µm, most preferably 10-40 µm. The average particle size is determined by laser method on Malvern Mastersizer.

In a preferred embodiment (a), wet granulation process comprises:
- providing rosiglitazone having defined average particle size (as defined above),
- granulating a mixture of excipients using alcohol, water/alcohol mixture or aqueous, alcoholic or aqueous/alcoholic granulating liquid to obtain granulate,
- addition of said rosiglitazone particles and excipient(s) to the granulate to give compression mixture,
- compressing the compression mixture to the desired form, and
- optionally, applying a coating.

In another preferred embodiment (b), wet granulation process comprises:
- providing rosiglitazone having defined average particle size (as defined above),
- granulating a mixture of said rosiglitazone and excipient(s), using alcohol, water/alcohol mixture or aqueous, alcoholic or aqueous/alcoholic granulating liquid to obtain granulate,
- addition of excipient(s) to the granulate to give compression mixture,
- compressing the compression mixture to the desired form, and
- optionally, applying a coating.

In another preferred embodiment (c), wet granulation process comprises:
- providing rosiglitazone having defined average particle size (as defined above),
- granulating a mixture of portion of said rosiglitazone and excipient(s), using alcohol, water/alcohol mixture or aqueous, alcoholic or aqueous/alcoholic granulating liquid to obtain granulate,
- addition of the rest of the said rosiglitazone and excipient(s) to the granulate to give compression mixture,
- compressing the compression mixture to the desired form, and
- optionally, applying a coating.

In embodiment (a), a granulate is prepared without including rosiglitazone thereto. In embodiment (b), a granulate is prepared which includes rosiglitazone. In another embodiment (c), rosiglitazone is divided between granulate and extragranular phase.

In embodiments (a), (b) and (c), granulating liquid can contain a solid substance which is dispersed, suspended or dissolved in the diluent. The dispersed substance can have known functions of excipients used in the present compositions comprising rosiglitazone, e.g. binder, surfactant or acidic agent.

According to a preferred embodiment, the present compounds are used for the preparation of a pharmaceutical composition for the prevention and/or treatment of a diabetic disease or inflammations. Preferably, the diabetic disease is non-insulin dependent diabetes, i.e. type II diabetes.

The following examples illustrate the invention and are not intended to restrict the scope of the invention in any way.

### Examples

The compounds according to the present invention were characterized with regard to their meltings points (Tm) by means of a Koffler melting point apparatus with an accuracy of approsimately ±1 °C and X-ray powder diffraction patterns (obtained by a Phillips PW3040/60 X'Pert PRO diffractometer using CuK_{α} radiation of 1,541874 Å). Images of particles were taken on a Microscope Olympus BX 50 equipped with Olympus camera DP70.

### Rosiglitazone Form I

### Example 1:

1.04 g (1mmol) of rosiglitazone base are added to 4 ml of 1,4-dioxane and the mixture is heated until all rosiglitazone is dissolved. The solution is filtered and cooled to 0°C. The precipitate formed is filtered and dried under room temperature. 0.5 g of rosiglitazone of Form I is isolated.
Tm: 153-155 °C
XRD: Form I

### Example 2:

1.04 g (1mmol) of rosiglitazone base are added to 17 ml of ethyl acetate and the mixture is heated until all rosiglitazone is dissolved. The solution is filtered and cooled to room temperature. The precipitate formed is filtered and dried under room temperature. 0.87 g of rosiglitazone of Form I is isolated.
Tm: 154-155°C
XRD: Form I

### Example 3:

1.04 g (1mmol) of rosiglitazone base are added to 4 ml of THF and the mixture is heated until all rosiglitazone is dissolved. The solution is filtered and cooled to room temperature. The precipitate formed is filtered and dried under room temperature. 0.5 g of rosiglitazone of Form I is isolated.
Tm: 152-154 °C
XRD: Form I

### Example 4:

1.04 g (1mmol) of rosiglitazone base are added to 20 ml of isopropyl acetate and the mixture is heated until all rosiglitazone is dissolved. The solution is cooled to 0°C and the precipitate formed is filtered and dried under room temperature. 0.97 g of rosiglitazone of Form I is isolated.
Tm: 149-153 °C
XRD: Form I

### Example 5:

1.04 g (1mmol) of rosiglitazone base are added to 54 ml of methanol and the mixture is heated until all rosiglitazone is dissolved. The solution is filtered and cooled to 0°C. The precipitate formed is filtered and dried under room temperature. 0.91 g of rosiglitazone of Form I is isolated.
Tm: 151-154 °C
XRD: Form I

### Example 6:

1.04 g (1mmol) of rosiglitazone base are added to 11 ml of 4-methyl-2-pentanone and the mixture is heated until all rosiglitazone is dissolved. The solution is filtered and cooled to 0°C. The precipitate formed is filtered and dried under room temperature. 0.95 g of rosiglitazone of Form I is isolated.
Tm: 152-154 °C
XRD: Form I

### Example 7:

1.04 g (1mmol) of rosiglitazone base are added to 26 ml of methyl acetate and the mixture is heated until all rosiglitazone is dissolved. The solution is filtered and cooled to 0°C. The precipitate formed is filtered and dried under room temperature. 0.89 g of rosiglitazone of Form I is isolated.
Tm: 154-156 °C
XRD: Form I

### Example 8:

1.04 g (1mmol) of rosiglitazone base are added to 14 ml of acetonitrile and the mixture is heated until all rosiglitazone is dissolved. The solution is filtered and cooled to 0°C. The precipitate formed is filtered and dried under room temperature. 0.98 g of rosiglitazone of Form I is isolated.
Tm:153-154°C
XRD: Form I

### Example 9:

1.04 g (1mmol) of rosiglitazone base are added to 4 ml of THF and the mixture is heated until all rosiglitazone is dissolved. To the solution 1 ml of water is added and the mixture is cooled to 0°C. The precipitate formed is filtered, dried under room temperature and 0.67 g of rosiglitazone of Form I is isolated.
Tm: 152-155 °C
XRD: Form I

### Example 10:

1.04 g (1mmol) of rosiglitazone base are added to 4 ml of THF and the mixture is heated until all rosiglitazone is dissolved. To the solution 4ml of tert-butyl methyl ether are added and the mixture is cooled to 0°C. The precipitate formed is filtered, dried under room temperature and 0.85 g of rosiglitazone of Form I is isolated.
Tm: 152-154 °C
XRD: Form I

### Example 11:

1.04 g (1mmol) of rosiglitazone base are added to 17 ml of ethyl acetate and the mixture is heated until all rosiglitazone is dissolved. To the solution 0.85 ml of water is added and the mixture is cooled to 0°C. The precipitate formed is filtered, dried under room temperature and 0.88 g of rosiglitazone of Form I is isolated.
T150-154 °C
XRD: Form I

### Example 12:

7 g (7.3mmol) of rosiglitazone base are added to 28 ml of THF and the mixture is heated until all rosiglitazone is dissolved. To the solution 7 ml of water is added and the solution is lyophilized and 7 g of rosiglitazone of Form I is isolated.
Tm: 146-152°C
XRD: Form I

### Rosiglitazone Form II

### Example 13:

1.04 g (1mmol) of rosiglitazone base are added to 4 ml of THF and the mixture is heated until all rosiglitazone is dissolved. To the solution 4ml of heptane are added and the mixture is cooled to 0°C. The precipitate formed is filtered, dried under room temperature and 0.85 g of rosiglitazone of Form II is isolated.
Tm: 153-154 °C
XRD: Form II

### Example 14:

1.04 g (1mmol) of rosiglitazone base are added to 4 ml of THF and the mixture is heated until all rosiglitazone is dissolved. To the solution 4ml of hexane is added and the mixture is cooled to 0°C. The precipitate formed is filtered, dried under room temperature and 0.97 g of rosiglitazone of Form II is isolated.
Tm: 152-154 °C
XRD: Form II

### Example 15:

1.04 g (1mmol) of rosiglitazone base are added to 4 ml of THF and the mixture is heated until all rosiglitazone is dissolved. To the solution 4ml of cyclohexane are added and the mixture is cooled to 0°C. The precipitate formed is filtered, dried under room temperature and 0.93 g of rosiglitazone of Form II is isolated.
Tm: 151-154 °C
XRD: Form II

### Example 16:

1.04 g (1mmol) of rosiglitazone base are added to 4 ml of THF and the mixture is heated until all rosiglitazone is dissolved. The solution is filtered and cooled below 0°C in 10 min. The precipitate formed is filtered and dried under room temperature. 0.5 g of rosiglitazone of Form II is isolated.
Tm: 152-154 °C
XRD: Form II

### Rosiglitazone Form III

### Example 17:

1.04 g (1mmol) of rosiglitazone base are suspended in 15 ml of water and 3.4 ml of 1M NaOH is added. Then the formed solution is neutralized with 1 M HCl and the precipitate formed is filtered, dried under room temperature and 0.96 g of rosiglitazone of Form III is isolated.
Tm: 140-148 °C
XRD: Form III

### Example 18:

1.04 g (1mmol) of rosiglitazone base is suspended in 15 ml of water and 3.9 ml of 1M HCl is added. Then the formed solution is neutralized with 1 M NaOH and the precipitate formed is filtered, dried under room temperature and 0.93 g of rosiglitazone of Form III is isolated.
Tm: 144-149°C
XRD: Form III

### Examples of pharmaceutical compositions comprising polymorphic forms I, II and III of rosiglitazone base

### Example 19

16 g of rosiglitazone, 8 g of sodium starch glycollate, 8 g of hydroxypropylmethylcellulose, 32 mg of microcrystalline cellulose and 136 g of lactose monohydrate were homogenised in high-shear mixer.

70 g of purified water was sprayed onto the mixture of rosiglitazone and excipients as listed above. The granulate obtained was dried in fluid-bed dryer at inlet air temperature 60°C. Loss on drying (measurement perfomed on apparatus Mettler Toledo HR73 halogen moisture analyser at 85°C for 20 minutes at 85°C for 20 minutes) of the sieved granulate was 0.80% by weight.

22 g of sodium starch glycollate, 87 g of microcrystalline cellulose and 288 g of lactose monohydrate were added to the granulate obtained above and mixed in a high-shear mixer. Finally, 3 g of magnesium stearate was added to obtain compression mixture. Loss on drying (measurement perfomed on apparatus Mettler Toledo HR73 halogen moisture analyser at 85°C for 20 minutes at 85°C for 20 minutes) of the compression mixture was 2.96% by weight.

The compression mixture was compressed into cores with theoretical weight of 300 mg.

Cores were coated in an automatic coating pan with water-based film coating suspension of ready-to-make mixtured, commercially available as Opadry. Theoretical weight of the film coated tablets was 309 mg.

### Example 20

16 g of rosiglitazone, 8 g of sodium starch glycollate, 8 g of hydroxypropylmethylcellulose, 32 mg of microcrystalline cellulose and 136 g of lactose monohydrate were homogenised in high-shear mixer.

5 g od sodium laurysulphate was dispersed in 60 g of purified water to obtain granulating liquid. Granulating liquid was sprayed onto the mixture of rosiglitazone and excipients as listed above. The granulate obtained was dried in fluid-bed dryer at inlet air temperature 60°C. Loss on drying (measurement perfomed on apparatus Mettler Toledo HR73 halogen moisture analyser at 85°C for 20 minutes at 85°C for 20 minutes) of the sieved granulate was 0.88% by weight.

22 g of sodium starch glycollate, 87 g of microcrystalline cellulose and 283 g of lactose monohydrate were added to the granulate obtained above and mixed in a high-shear mixer. Finally, 3 g of magnesium stearate was added to obtain compression mixture. Loss on drying (measurement perfomed on apparatus Mettler Toledo HR73 halogen moisture analyser at 85°C for 20 minutes at 85°C for 20 minutes) of the compression mixture was 0.99% by weight.

The compression mixture was compressed into cores with theoretical weight of 300 mg.

Cores were coated in an automatic coating pan with water-based film coating suspension of ready-to-make mixture as described in Example 19.

### Example 21

16 g of rosiglitazone, 7 g of tartaric acid, 8 g of sodium starch glycollate, 8 g of hydroxypropylmethylcellulose, 32 mg of microcrystalline cellulose and 136 g of lactose monohydrate were homogenised in high-shear mixer.

50 g of purified water was sprayed onto the mixture of rosiglitazone and excipients as listed above. The granulate obtained was dried in fluid-bed dryer at inlet air temperature 60°C. Loss on drying (measurement perfomed on apparatus Mettler Toledo HR73 halogen moisture analyser at 85°C for 20 minutes at 85°C for 20 minutes) of the sieved granulate was 0.98% by weight.

22 g of sodium starch glycollate, 87 g of microcrystalline cellulose and 281 g of lactose monohydrate were added to the granulate obtained above and mixed in a high-shear mixer. Finally, 3 g of magnesium stearate was added to obtain compression mixture. Loss on drying (measurement perfomed on apparatus Mettler Toledo HR73 halogen moisture analyser at 85°C for 20 minutes at 85°C for 20 minutes) of the compression mixture was 1.00% by weight.

The compression mixture was compressed into cores with theoretical weight of 300 mg.

Cores were coated in an automatic coating pan with water-based film coating suspension of ready-to-make mixture as described in Example 19.

### Example 22

16 g of rosiglitazone, 6 g of asparaginic acid, 8 g of sodium starch glycollate, 8 g of hydroxypropylmethylcellulose, 32 mg of microcrystalline cellulose and 136 g of lactose monohydrate were homogenised in high-shear mixer.

63 g of purified water was sprayed onto the mixture of rosiglitazone and excipients as listed above. The granulate obtained was dried in fluid-bed dryer at inlet air temperature 60°C. Loss on drying (measurement perfomed on apparatus Mettler Toledo HR73 halogen moisture analyser at 85°C for 20 minutes at 85°C for 20 minutes) of the sieved granulate was 0.70% by weight.

22 g of sodium starch glycollate, 87 g of microcrystalline cellulose and 282 g of lactose monohydrate were added to the granulate obtained above and mixed in a high-shear mixer. Finally, 3 g of magnesium stearate was added to obtain compression mixture. Loss on drying (measurement perfomed on apparatus Mettler Toledo HR73 halogen moisture analyser at 85°C for 20 minutes at 85°C for 20 minutes) of the compression mixture was 0.89% by weight.

The compression mixture was compressed into cores with theoretical weight of 300 mg.

Cores were coated in an automatic coating pan with water-based film coating suspension of ready-to-make mixture as described in Example 19.

## Claims

1. Polymorphic form I of rosiglitazone base, **characterized by** a X-ray powder diffraction pattern exhibiting characteristic diffractions at 13,9;16,6; 17,3; 21,9; 22,4; 25,3; 27,5 and 30,0° ± 0,2°2Theta.

2. Polymorphic form II of rosiglitazone base, **characterized by** a X-ray powder diffraction pattern exhibiting characteristic diffractions at 14,2; 15,9; 17,1;17,6; 21,3; 22,1; 22,8; 26,0 and 26,1° ± 0,2°2Theta.

3. Polymorphic form III of rosiglitazone base, **characterized by** a X-ray powder diffraction pattern exhibiting characteristic diffractions at 13,8; 16,5; 17,2; 21,7; 22,1; 22,8; 25,0; 27,7 and 28,5° ± 0,2°2Theta.

4. Process for the preparation of Polymorphic form I of rosiglitazone base, comprising the steps of:
- dissolving rosiglitazone base in an organic solvent at a temperature in the range of from 20°C to reflux temperature of said organic solvent;
- optionally adding an anti-solvent;
- cooling to a temperature in the range of from -10°C to 30°C; and
- separating and drying of Polymorphic form I of rosiglitazone base; wherein
the organic solvent is selected from the group consisting of an alcohol, an ester, an ether, a ketone and THF; and
the anti-solvent is selected from the group consisting of water and an organic antisolvent.

5. Process for the preparation of Polymorphic form I of rosiglitazone base, comprising the steps of:
- dissolving rosiglitazone base in an organic solvent at a temperature in the range of from 20°C to reflux temperature of said organic solvent;
- optionally adding water;
- lyophilising and drying; wherein
the organic solvent is selected from the group consisting of an alcohol, an ester and a ketone.

6. Process for the preparation of Polymorphic form II of rosiglitazone base, comprising the steps of:
- dissolving rosiglitazone base in an organic solvent at a temperature in the range of from 20°C to reflux temperature of said organic solvent;
- adding an anti-solvent and cooling to a temperature in the range of from -10°C to 30°C or cooling to a temperature in the range of from -10°C to 30°C at a rate of 1 to 30 °C/min. and stirring; and
- separating and drying of Polymorphic form II of rosiglitazone base; wherein the organic solvent is selected from the group consisting of tetrahydrofurane, dioxane, an alcohol, an ester and a ketone; and
the anti-solvent is selected from the group consisting of an organic antisolvent such as alkanes and cycloalkanes.

7. Process for the preparation of Polymorphic form II of rosiglitazone base, comprising the steps of:
- dissolving rosiglitazone base in an organic solvent at a temperature in the range of from 20°C to reflux temperature of said organic solvent; and
- evaporating said organic solvent under normal pressure or reduced pressure, wherein the organic solvent used is a ketone.

8. Process for the preparation of Polymorphic form III of rosiglitazone base, comprising the steps of:
- dissolving rosiglitazone base in an aqueous solution of an inorganic base at a temperature in the range of from 20°C to 100°C;
- adding an aqueous solution of an inorganic acid until neutralization;
- stirring at a temperature in the range of from 0°C to 20°C;
- separating and drying of Polymorphic form III of rosiglitazone base.

9. Process for the preparation of Polymorphic form III of rosiglitazone base, comprising the steps of:
- dissolving rosiglitazone base in an organic solvent at a temperature in the range of from 20°C to reflux temperature of said organic solvent; and
- evaporating said organic solvent under normal pressure or reduced pressure, wherein
the organic solvent used is selected from the group consisting of an alcohol, an ester, an ether, tetrahydrofurane and dioxane.

10. The process according to any of the claims 4-6 and 8, wherein the organic solvent is methanol.

11. Pharmaceutical composition comprising polymorphic form I, II or III of rosiglitazone base.

12. The pharmaceutical composition according to claim 11, which is in the form of tablets, pills, powders, lozenges, sacchets, soft/ hard gelatine capsules or suppositories.

13. Use of Polymorphic form I, II or III of rosiglitazone base in the preparation of a pharmaceutical composition for the prevention and/or treatment of a diabetic disease or inflammations.

14. The use according to claim 13, wherein the diabetic disease is non-insulin dependent diabetes.
